# EUROPEAN PATENT APPLICATION

(11) **EP 4 513 186 A1**
(43) Date of publication of application: **26.02.2025**
(21) Application number: 23792021.0
(22) Date of filing: 07.03.2023
(51) Int. Cl.: G01N 33/50, C12N 5/09

(54) **COMPOSITION FOR PREDICTING OXALIPLATIN DRUG EFFICACY, AND USE THEREOF**

(30) Priority: 19.04.2022 KR 20220048368
(71) Applicant: Samsung Life Public Welfare Foundation, Seoul 04348 (KR)
(72) Inventor: CHO, Yong Beom, Seoul 06351 (KR); SHIN, Yong Jae, Seoul 06351 (KR)
(74) Representative: Weickmann & Weickmann PartmbB
(86) International application number: PCT/KR2023/003074
(87) International publication number: WO 2023/204441

(57) **Abstract**

The present invention relates to a composition for predicting efficacy of oxaliplatin drug, and the use thereof. Using composition for predicting efficacy of oxaliplatin drug, it is possible to predict the responsiveness of a colorectal cancer patient to oxaliplatin treatment so that the patient may be treated with selected drugs suitable for the patient, thus increasing the treatment success rate.

## Description

### Technical Field

The present invention relates to a composition for predicting efficacy of oxaliplatin drug and the use thereof.

### Background Art

Colorectal cancer refers to a malignant tumor that occurs in the colon and rectum, and about 28,000 people are newly diagnosed with colorectal cancer every year. Fortunately, if colorectal cancer is detected early through regular endoscopic examinations, more than 90% can be cured and the 5-year survival rate is over 75%. Nevertheless, in many cases, colorectal cancer is detected in late stages or is not appropriately treated, and thus it is the third leading cause of cancer death in Korea.

For drug treatment of colorectal cancer, anticancer therapy is typically performed using FOLFIRI consisting of leucovorin, 5-fluorouracil (5-FU), and irinotecan, or FOLFOX consisting of leucovorin, 5-fluorouracil (5-FU), and oxaliplatin. The biggest difference between the two anticancer therapies lies in the use of irinotecan or oxaliplatin. 70% of colorectal cancer patients are sensitive to FOLFIRI containing irinotecan. Thus, currently, in the medical field, therapy for colorectal cancer consists of treatment with FOLFIRI, followed by additional treatment with FOLFOX depending on the prognosis.

However, if the prognosis is poor during this process, the cancer further progresses in many cases. In addition, even among patients with similar clinical characteristics and the same stage, the response to anticancer chemotherapy appears to vary, and thus predicting anticancer therapy suitable for each patient is an important issue in the treatment of colorectal cancer.

### DISCLOSURE

### Technical Problem

One aspect is to provide a composition for predicting efficacy of oxaliplatin drug comprising advanced DMEM/F12, L-glutamine, HEPES, an abtibiotic, B27 supplement, N2 supplement, N-acetylcysteine, nicotinamide, gastrin, and EGF.

Another aspect is to provide a method for predicting efficacy of oxaliplatin drug, comprising steps of: culturing organoids comprising colorectal cancer cells, advanced DMEM/F12, L-glutamine, HEPES, an abtibiotic, B27 supplement, N2 supplement, N-acetylcysteine, nicotinamide, gastrin, and EGF; treating the cultured organoids with oxaliplatin; and determining the sensitivity to oxaliplatin of the organoids treated with oxaliplatin.

### Technical Solution

One aspect provides a composition for predicting efficacy of oxaliplatin drug comprising advanced DMEM/F12, L-glutamine, HEPES, an abtibiotic, B27 supplement, N2 supplement, N-acetylcysteine, nicotinamide, gastrin, and EGF.

According to one embodiment, the antibiotic may comprise at least one of penicillin, streptomycin, or Primocin.

According to one embodiment, the composition may further comprise at least one of Y-27632, Wnt3a, or Noggin.

Another aspect is to provide a method for predicting efficacy of oxaliplatin drug, comprising steps of: culturing organoids comprising colorectal cancer-derived cells, advanced DMEM/F12, L-glutamine, HEPES, an abtibiotic, B27 supplement, N2 supplement, N-acetylcysteine, nicotinamide, gastrin, and EGF; treating the cultured organoids with oxaliplatin; and determining the sensitivity to oxaliplatin of the organoids treated with oxaliplatin.

According to one embodiment, the organoids in the step of culturing the organoids may further comprise at least one of Y-27632, Wnt3a, or Noggin.

According to one embodiment, the sensitivity to oxaliplatin may be determined by evaluating IC50, AUC, and cell viability.

### Advantageous Effects

Using the method for producing organoids, organoids, and method for predicting responsiveness of colorectal cancer patients to treatment according to the present invention, it is possible to predict responsiveness to oxaliplatin so that a patient may be treated with drugs suitable for the patient, thus reducing additional treatments and increasing the treatment success rate.

### Brief Description of Drawings

FIG. 1 schematically shows a method process for predicting efficacy of oxaliplatin drug.
FIG. 2 shows data comparing the cell viability following treatment with each of anticancer drugs oxaliplatin, 5-FU, irinotecan, and folinic acid after 3D cell culture with the cell viability of a colorectal cancer cell line.
FIG. 3 is a graph comparing the oxaliplatin resistance of colorectal cancer cell line HCT116 cells line in terms of AUC values depending on whether or not HCT116 cell-based organoid culture media contain inhibitors and activators.
FIG. 4 is a graph comparing the oxaliplatin resistance of organoids in terms of IC50 values depending on whether or not colorectal cancer cell line HCT116 cell-based organoid culture media contain inhibitors and activators.
FIG. 5 is a graph comparing the oxaliplatin resistance of organoids in terms of cell viability values versus oxaliplatin treatment concentration depending on whether or not colorectal cancer cell line HCT116 cell-based organoid culture media contain inhibitors and activators.
FIG. 6 is a graph comparing the oxaliplatin resistance of organoids in terms of AUC values depending on whether or not colorectal cancer cell line RKO cell-based organoid culture media contain inhibitors and activators.
FIG. 7 is a graph comparing the oxaliplatin resistance of organoids in terms of IC50 values depending on whether or not colorectal cancer cell line RKO cell-based organoid culture media contain inhibitors and activators.
FIG. 8 is a graph comparing the oxaliplatin resistance of organoids in terms of cell viability values versus oxaliplatin treatment concentration depending on whether or not colorectal cancer cell line RKO cell-based organoid culture media contain inhibitors and activators.
FIG. 9 is a graph comparing the oxaliplatin resistance of organoids in terms of AUC values depending on whether or not colorectal cancer cell line SW620 cell-based organoid culture media contain inhibitors and activators.
FIG. 10 is a graph comparing the oxaliplatin resistance of organoids in terms of IC50 values depending on whether or not colorectal cancer cell line SW620 cell-based organoid culture media contain inhibitors and activators.
FIG. 11 is a graph comparing the oxaliplatin resistance of organoids in terms of cell viability values versus oxaliplatin treatment concentration depending on whether or not colorectal cancer cell line SW620 cell-based organoid culture media contain inhibitors and activators.
FIG. 12 is a graph comparing the oxaliplatin resistance of organoids in terms of AUC values depending on whether or not colorectal cancer cell line LS147T cell-based organoid culture media contain inhibitors and activators.
FIG. 13 is a graph comparing the oxaliplatin resistance of organoids in terms of IC50 values depending on whether or not colorectal cancer cell line LS147T cell-based organoid culture media contain inhibitors and activators.
FIG. 14 is a graph comparing the oxaliplatin resistance of organoids in terms of cell viability values versus oxaliplatin treatment concentration depending on whether or not colorectal cancer cell line LS147T cell-based organoid culture media contain inhibitors and activators.
FIG. 15 is a graph comparing the oxaliplatin resistance of organoids in terms of AUC values depending on whether or not colorectal cancer cell line HT29 cell-based organoid culture media contain inhibitors and activators.
FIG. 16 is a graph comparing the oxaliplatin resistance of organoids in terms of cell viability values versus oxaliplatin treatment concentration depending on whether or not colorectal cancer cell line HT29 cell-based organoid culture media contain an inhibitor and an activator.
FIG. 17 is a graph comparing the oxaliplatin resistance of organoids in terms of AUC values depending on whether or not colorectal cancer cell line HCT15 cell-based organoid culture media contain inhibitors and activators.
FIG. 18 is a graph comparing the oxaliplatin resistance of organoids in terms of cell viability values versus oxaliplatin treatment concentration depending on whether or not colorectal cancer cell line HCT15 cell-based organoid culture media contain inhibitors and activators.
FIG. 19 is a graph comparing the oxaliplatin resistance of organoids in terms of AUC values depending on whether or not colorectal cancer cell line CoLo205 cell-based organoid culture media contain inhibitors and activators.
FIG. 20 is a graph comparing the oxaliplatin resistance of organoids in terms of cell viability values versus oxaliplatin treatment concentration depending on whether or not colorectal cancer cell line CoLo205 cell-based organoid culture media contain inhibitors and activators.
FIG. 21 is a graph comparing the oxaliplatin resistance of organoids in terms of AUC values depending on whether or not colorectal cancer cell line LoVo cell-based organoid culture media contain inhibitors and activators.
FIG. 22 is a graph comparing the oxaliplatin resistance of organoids in terms of cell viability values versus oxaliplatin treatment concentration depending on whether or not colorectal cancer cell line LoVo cell-based organoid culture media contain inhibitors and activators.
FIG. 23 is a graph comparing the oxaliplatin resistance change of organoids in terms of AUC values depending on whether or not media for culture of organoids based on colorectal cancer cell lines (HCT116, RKO, SW620, and LS147T) highly sensitive to oxaliplatin contain inhibitors and activators (Ex1 and Ex2).
FIG. 24 is a graph comparing the oxaliplatin resistance change of organoids in terms of AUC values depending on or not whether media for culture of organoids based on colorectal cancer cell lines (HT29, HCT15, CoLo205, and LoVo) showing low sensitivity to oxaliplatin contain inhibitors and activators (Ex1 and Ex2).
FIG. 25 shows data showing the results of evaluating the drug responsiveness of HCT-116 colorectal cancer cell line, an organoid cultured in Ex1, and an organoid cultured in Ex2, which are sensitive to oxaliplatin, after treatment with each of oxaliplatin, 5-FU, irinotecan, and folinic acid.
FIG. 26 depicts data showing the results of evaluating how cell viability varies depending on oxaliplatin treatment concentration in the case in which the culture in media for culture of 166T colorectal cancer patient organoids, which contain or not contain inhibitors and activators (Ex1 and Ex2), is two-dimensional culture (A) or three-dimensional culture (B).
FIG. 27 depicts data showing the results of evaluating and comparing changes in AUC and IC50 values after two-dimensional culture and three-dimensional culture depending on whether or not media for culture of 166T colorectal cancer patient-derived organoids contain an inhibitor and an activator (Ex1 and Ex2).
FIG. 28 shows data showing the results of comparing the expression levels of E-cadherin and EpCAM day 5 after organoid production with the expression levels in existing organoids (Ex1) containing both inhibitors and activators, in order to evaluate whether organoids (Ex2) produced using organoid culture media without inhibitors and activators retain organoid characteristics.
FIG. 29 shows data showing the results of comparing the IC50 value of oxaliplatin in organoid culture conditions containing both inhibitors and activators (Ex1), organoid culture conditions without both inhibitors and activators (Ex2), and organoid culture conditions containing only one of inhibitors and activators (Ex2-1 to Ex2-6), in order to determine which factor among the inhibitors and the activators is involved in oxaliplatin resistance.
FIG. 30 shows data showing the results of comparing the AUC values for oxaliplatin in organoids containing both inhibitors and activators (Ex1), organoids without both inhibitors and activators (Ex2), and organoids containing only one of inhibitors and activators (Ex2-1 to Ex2-6), in order to determine which factor among the inhibitors and the activators is involved in oxaliplatin resistance.

### Best Mode

One aspect provides a composition for predicting efficacy of oxaliplatin drug comprising advanced DMEM/F12, L-glutamine, HEPES, an abtibiotic, B27 supplement, N2 supplement, N-acetylcysteine, nicotinamide, gastrin, and EGF.

The advanced DMEM/F12 may be a basal medium that is used to culture animal cells.

The L-glutamine is a component that is used for protein synthesis in media and as a major energy source in media to affect cell concentration and proliferation rate, and may be one of the amino acids that are essential components of media.

The HEPES acts as a zwitterionic organic chemical buffer in media and may be a component that maintains physiological pH despite changes in carbon dioxide concentration.

The B27 supplement and N2 supplement may be components that promote differentiation and proliferation of cells in media.

The N-acetylcysteine may be a component that supplies cysteine into cells to activate the biosynthesis of glutathione, an antioxidant biomolecule, thereby exhibiting an antioxidant effect. The component may also be a component that promotes differentiation and proliferation of cells in media.

The nicotinamide is a cell culture supplement and may be a necessary component for cell differentiation.

The gastrin may be a mitotic factor for epithelial cells.

The EGF is a physiologically active protein and may be a component that affects cell differentiation.

According to one embodiment, the antibiotic may comprise at least one of penicillin, streptomycin, or Primocin.

The antibiotic may be a component that is used to selectively culture only the cells to be cultured.

According to one embodiment, the composition may further comprise at least one of Y-27632, Wnt3a, or Noggin.

The Y-27632 is a cell-penetrating component and may be a ROCK (Rho-associated, coiled-coil containing protein kinase) inhibitor.

The Wnt3a may be an essential component in maintaining the proliferation of benign stem cells in organoids.

The Noggin may be a necessary component for maintaining human embryonic stem cells that do not differentiate *in vitro,* and may be a component that prevents short term spontaneous differentiation of cells.

The oxaliplatin is a type of platinum-based anticancer drug, and may be a substance that inhibits DNA repair and DNA synthesis through DNA cross-linking.

Since the composition is cultured with colorectal cancer-derived cells, it may predict the efficacy of oxaliplatin drug.

The colorectal cancer refers to a malignant tumor that occurs in the colon, and is classified as a malignant pathological condition based on, for example, colonoscopic surveillance and biopsy tissue.

The colorectal cancer-derived cells may be cells derived from adenocarcinoma, malignant carcinoid, leiomyosarcoma, Kaposi's sarcoma, etc., without being limited thereto.

The colorectal cancer-derived cells may be cells of HCT116, RKO, SW620, LS147T, HT29, HTC15, CoLo205, and LoVo cell lines, without being limited thereto.

The "predicting efficacy of drug" refers to the act of a patient predicting the course and outcome of a disease with respect to a drug or drug set. For example, the course of a disease after treatment may vary depending on the patient's physiological or environmental condition, and the "predicting efficacy of drug" may refer to any action that predicts the course of the disease after treatment by comprehensively considering the patient's condition, and may refer to an action of predicting drug responsiveness.

The drug responsiveness refers to the degree of effectiveness of drug treatment for an individual, for example, an individual suffering from a cancer disease. For example, the term "increased responsiveness" or "good responsiveness" when used in connection with the treatment of a cancer patient may refer to an increase in drug effectiveness when measured using any method known in the art. As another example, the responsiveness of a cancer patient to the drug may be characterized by complete or partial response. As still another example, the increased responsiveness of a cancer patient to the drug may be characterized by overall survival rate, disease-free survival, target response rate, time to tumor progression, progression-free survival, or time to treatment failure.

The composition for predicting efficacy of oxaliplatin drug has weakened resistance to oxaliplatin which increases as the composition is formed into organoids. The composition may be cultured similar to the characteristics of cells that are before three-dimensional culture.

In one example, it was shown that, when the colorectal cancer-derived cells of the composition for predicting efficacy of oxaliplatin drug were sensitive to oxaliplatin, their resistance to oxaliplatin was reduced, indicating low AUC and IC50 values of the cells, suggesting that their sensitivity to oxaliplatin was high. On the other hand, it was confirmed that, when the colorectal cancer-derived cells had low sensitivity to oxaliplatin, the AUC and IC50 values of the cells were still high, indicating that that the sensitivity of the cells to oxaliplatin was low.

According to one embodiment, the composition for predicting efficacy of oxaliplatin drug may be in the form of an organoid. The term "organoid" in the present specification refers to cells having a 3D structure, and refers to a colorectal cancer-like model produced through an artificial culture process without being collected or obtained from an animal or the like. The origin of the cells that make up the organoid is not limited. Unlike 2D culture, 3D cell culture allows cells to grow in all directions *in vitro.* The organoids may be used to develop therapeutic agents for diseases by closely mimicking organs interacting *in vivo.*

In one example, it was confirmed that the composition for predicting efficacy of oxaliplatin drug showed similar results even in two-dimensional culture, but when 3D cultured organoids were prepared from the composition, it was easier to predict the efficacy of oxaliplatin drug using 3D cultured organoids compared to other organoids that showed resistance to oxaliplatin.

Another aspect is to provide a method for predicting efficacy of oxaliplatin drug, comprising steps of: culturing organoids comprising colorectal cancer-derived cells, advanced DMEM/F12, L-glutamine, HEPES, an abtibiotic, B27 supplement, N2 supplement, N-acetylcysteine, nicotinamide, gastrin, and EGF; treating the cultured organoids with oxaliplatin; and determining the sensitivity to oxaliplatin of the organoids treated with oxaliplatin.

Contents regarding the colorectal cancer, colorectal cancer-derived cells, advanced DMEM/F12, L-glutamine, HEPES, antibiotic, B27 supplement, N2 supplement, N-acetylcysteine, nicotinamide, gastrin, EGF, organoids, oxaliplatin, predicting efficacy of drug, and composition for predicting efficacy of oxaliplatin drug are the same as those described above.

According to one embodiment, the organoids in the step of culturing the organoids may further comprise at least one of Y-27632, Wnt3a, or Noggin.

Contents regarding the Y-27632, Wnt3a, and Noggin are the same as those described above.

According to one embodiment, the sensitivity to oxaliplatin may be determined by evaluating IC50, AUC, and cell viability.

The culturing refers to culturing cells isolated from a tissue of an organism according to a known method depending on the type of cell. In addition, the culturing includes any action that can produce or maintain organoids.

Determining the sensitivity may be performed according to a known method, and the method for determining the sensitivity is not limited. For example, when activity or toxicity is tested using colorectal cancer organoids, the testing may be performed by a method of checking cell viability or measuring drug metabolism activity.

For example, as the standard for confirming that cells are sensitive to the drug, the response of the cells to the drug is assumed to be 100%, and the luminescence value of surviving cells after drug treatment may be analyzed and compared with the quantified drug response value.

In one example of the present invention, the average AUC value of colorectal cancer cell lines was 408.05 in Ex1, while the average AUC value was 384.32 in Ex2. In addition, it was confirmed that the average AUC value of the sensitive colorectal cancer cell lines was 396.08 in Ex1, while the average AUC value was 347.78 in Ex2. On the other hand, it was confirmed that the average AUC value of non-sensitive colorectal cancer cell lines was 420.02 in Ex1, while the average AUC value was 420.86 in Ex2. Therefore, when the AUC of cells is equal to or lower than the AUC of Ex2 (384.32) or the difference between Ex2 and Ex1 is 23.73 or more, which is the average, the cells can be determined to be sensitive to the drug. Here, the value 23.73 represents the difference between the average AUC of cells lines in Ex1 and the average AUC of the cell lines in Ex2.

In addition, referring to other papers [Accuracy of Using a Patient-Derived Tumor Organoid Culture Model to Predict the Response to Chemotherapy Regimens In Stage IV Colorectal Cancer: A Blinded Study, DISEASES OF THE COLON & RECTUM VOLUME 64: 7 (2021)], the condition that IC50 is 10 µM or less may be used as an aid in determining the sensitivity.

### Mode for Invention

One or more embodiments will be described in more detail below by way of examples. However, these examples are intended to illustrate one or more embodiments and the scope of the present invention is not limited to these examples.

### Experimental Example 1. Preparation of Organoid Culture Medium to be Treated with Drug

### 1-1. Organoid Culture Stage

Advanced DMEM/F12+++ (hereinafter referred to as "Ad/DF+++") was used to produce colorectal cancer organoids. Ad/DF+++ was refrigerated at 4°C until use, and was used in a cold state during cell washing. Ad/DF+++ medium containing the reagents shown in Table 1 below along with Ad/DF 1x (Gibco) was prepared.

**[Table 1]**

| Reagent | **Manufacturer** | **Cat #** | **Volume** |
|---|---|---|---|
| **Advanced DMEM/F12** | Gibco | 12634-028 | 1x |
| **Glutamax** | Gibco | 35050-061 | 1x |
| **HEPES** | Gibco | 15630-080 | 1x |
| **Penicillin / Streptomycin** | Gibco | 15140-122 | 1x |
| **Primocin** | Invivogen | ant-pm-2 | 100 µg/ml |

After preparing the Ad/DF+++ medium containing the above reagents, an appropriate amount of Matrigel (Corning #356231) was added so that the ratio between Matrigel and cells suspended in the Ad/DF+++ medium was 7:3. Then, the cells were plated on low attachment well plates at an appropriate density. The density and the type and number of well plates were determined according to the characteristics of each cell type. The medium was prepared according to the composition shown in Table 2 below, and the cells were cultured (expanded) until the required level was reached while the medium was replaced every 3 to 4 days.

**[Table 2]**

| Component | **Ex1** |
|---|---|
| **Advanced DMEM/F12** | 1x |
| **Glutamax** | 1x |
| **HEPES** | 1x |
| **Penicillin / Streptomycin** | 1x |
| **Primocin** | 100 µg/ml |
| **B27 supplement** | 1x |
| **N2 supplement** | 1x |
| **N-Acetylcysteine** | 1 mM |
| **Nicotinamide** | 10 mM |
| **Gastrin** | 10 nM |
| **EGF** | 10 ng/ml |
| **A83-01** | 500 nM |
| **Y-27632** | 10 µM |
| **SB 202190** | 10 µM |
| **Wnt3a** | 100 ng/ml |
| **R-spondin 1** | 1 µg/ml |
| **Noggin** | 100 ng/ml |

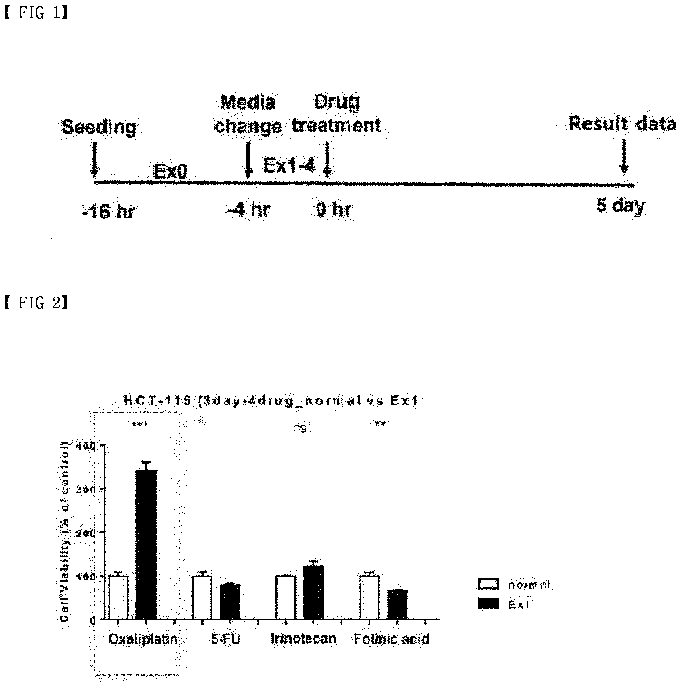

After producing the organoids as described above, the experiment was performed in the same order as in FIG. 1.

### 1-2. Seeding Step

If the organoids were expanded as needed for the experiment, the medium was carefully removed, taking care not to touch the Matrigel. The cells were separated from the Matrigel by pipetting about 20 times with 1 ml of cold Ad/DF+++. The cells in 1 to 4 wells per 6-well plate were collected in one 50 ml tube. If cells are collected from more than 4 wells into one tube, the Metrigel may not be washed properly. For this reason, the cells were placed in several tubes. Thereafter, Ad/DF+++ was poured to 20 to 50 ml of each tube, followed by centrifugation at 400g for 3 minutes at 4°C. The cells were dissociated with 1 to 2 mL of TrypLE^{™} Express Enzyme (Gibco, 1264-013), transferred to low-attachment 24 well plates, and then treated with 10 µM of Y-27632. Next, the cells were cultured in a CO₂ incubator for 5 minutes at 37°C. After pipetting about 20 times, whether the cells would be dissociated into single cells was checked by microscopic observation. If the cells were not dissociated into single cells, the culture under CO₂ and pipetting processes were repeated. After the single cells were transferred into 15-ml tubes, Ad/DF+++ was filled to 10 to 15 ml of each tube, followed by centrifugation at 400g at 4°C for 3 minutes. After suspending the pellet in Ad/DF +++, the cells were counted and diluted with medium according to the required number of cells.

### 1-3. Medium Change Step

12 hours after seeding in Experimental Example 1-2 above, the medium was changed with the medium shown in Table 3 below, followed by culturing to produce organoids.

**[Table 3]**

| Component | **Ex1** | **Ex2** |
|---|---|---|
| **Advanced DMEM/F12** | 1x | 1x |
| **Glutamax** | 1x | 1x |
| **HEPES** | 1x | 1x |
| **Penicillin / Streptomycin** | 1x | 1x |
| **Primocin** | 100 µg/ml | 100 µg/ml |
| **B27 supplement** | 1x | 1x |
| **N2 supplement** | 1x | 1x |
| **N-Acetylcysteine** | 1 mM | 1 mM |
| **Nicotinamide** | 10 mM | 10 mM |
| **Gastrin** | 10 nM | 10 nM |
| **EGF** | 10 ng/ml | 10 ng/ml |
| **A83-01** | 500 nM | - |
| **Y-27632** | 10 µM | - |
| **SB 202190** | 10 µM | - |
| **Wnt3a** | 100 ng/ml | - |
| **R-spondin 1** | 1 µg/ml | - |
| **Noggin** | 100 /ml | - |

### 1-4. Drug Treatment and Evaluation of Result Data

Four hours after the medium change, the cells were treated with oxaliplatin, 5-FU, Irinotecan, and folinic acid. Five days after drug treatment, intracellular ATP expression was determined using the ATPlite assay kit (PerkinElmer), and the data were expressed in terms of cell viability, IC50, and AUC.

### 1-5. Determination of Sensitivity to Oxaliplatin

As the standard for confirming that cells are sensitive to the drug, the response of the cells to the drug was assumed to be 100%, and the luminescence value of surviving cells after drug treatment was analyzed and compared with the quantified drug response value.

### Example 1. Checking of Increased Resistance to Oxaliplatin

The colorectal cancer cell line HCT-116 and the HCT-116 cells cultured under the organoid culture conditions of Ex1 known to be used to produce organoids was treated with each of oxaliplatin, 5-FU, irinotecan, and folinic acid.

As a result, as shown in FIG. 2, it was confirmed that, when treated with 5-FU, irinotecan, and folinic acid, the colorectal cancer cell line showed drug response results similar to those of the cells cultured under the organoids culture conditions, but when treated with oxaliplatin, the cells cultured under the organoids culture conditions of Ex1 proliferated compared to the colorectal cancer cell line. Thus, it was confirmed that, when treated with oxaliplatin, the colorectal cancer cells have increased resistance under the organoid culture conditions.

### Example 2. Confirmation of Organoids Having Reduced Resistance to Oxaliplatin

Based on the assumption that the factor that increases the resistance of the organoids to oxaliplatin is an activator or inhibitor that changes the phenotype, the organoids prepared by excluding some or all of activators and inhibitors as shown in Table 4 below were treated with oxaliplatin.

**[Table 4]**

| | **Ex1** | **Ex2** | **Ex3** | **Ex4** |
|---|---|---|---|---|
| **Advanced DMEM/F12** | 1x | 1x | 1x | 1x |
| **Glutamax** | 1x | 1x | 1x | 1x |
| **HEPES** | 1x | 1x | 1x | 1x |
| **Penicillin / Streptomycin** | 1x | 1x | 1x | 1x |
| **Primocin** | 100 µg/ml | 100 µg/ml | 100 µg/ml | 100 µg/ml |
| **B27 supplement** | 1x | 1x | 1x | 1x |
| **N2 supplement** | 1x | 1x | 1x | 1x |
| **N-Acetylcysteine** | 1 mM | 1 mM | 1 mM | 1 mM |
| **Nicotinamide** | 10 mM | 10 mM | 10 mM | 10 mM |
| **Gastrin** | 10 nM | 10 nM | 10 nM | 10 nM |
| **EGF** | 10 ng/ml | 10 ng/ml | 10 ng/ml | 10 ng/ml |
| **A83-01** | 500 nM | - | 500 nM | - |
| **Y-27632** | 10 µM | - | 10 µM | - |
| **SB 202190** | 10 µM | - | 10 µM | - |
| **Wnt3a** | 100 ng/ml | - | - | 100 ng/ml |
| **R-spondin 1** | 1 µg/ml | - | - | 1 µg/ml |
| **Noggin** | 100 ng/ml | - | - | 100 ng/ml |

In order to confirm reduced resistance to oxaliplatin, HCT116, RKO, SW620, and LS147T cell lines, which are highly sensitive to oxaliplatin, and HT29, HTC15, CoLo205, and Lovo cell lines, which are low sensitive to oxaliplatin, were used.

### 2-1. Evaluation of Resistance Reduction Effect in HCT116 Cells

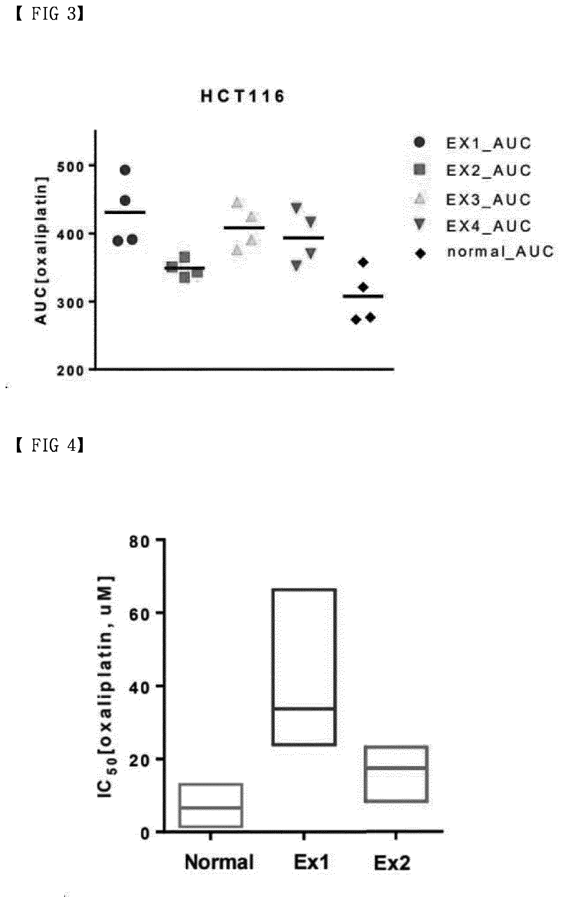

The HCT116 cell line sensitive to oxaliplatin was used and cultured in the organoid culture media shown in Table 4 above. As a result of treating the cell line and the cells cultured in Ex1 to Ex4 with oxaliplatin, as shown in FIG. 3, it was confirmed that Ex3 and Ex4, which do not contain inhibitors or activators factors, showed lower AUC values than Ex1, and the organoid culture conditions of Ex2 not containing both inhibitors and activators showed significantly lower AUC values than those of Ex1. This means that the sensitivity to oxaliplatin increased, and specifically, means that when the HCT116 cell line was cultured under the culture conditions of Ex2, the degree of increased resistance thereof to oxaliplatin decreased compared to that in the existing organoid culture conditions (Ex1).

To clearly compare them, the IC50 was measured after the HCT116 cell line and the organoids Ex1 and Ex2 with oxaliplatin. As a result, as shown in FIG. 4, it was confirmed that the IC50 value of the HCT116 cell line was similarly lowered in Ex2 compared to Ex1, which had increased resistance to oxaliplatin.

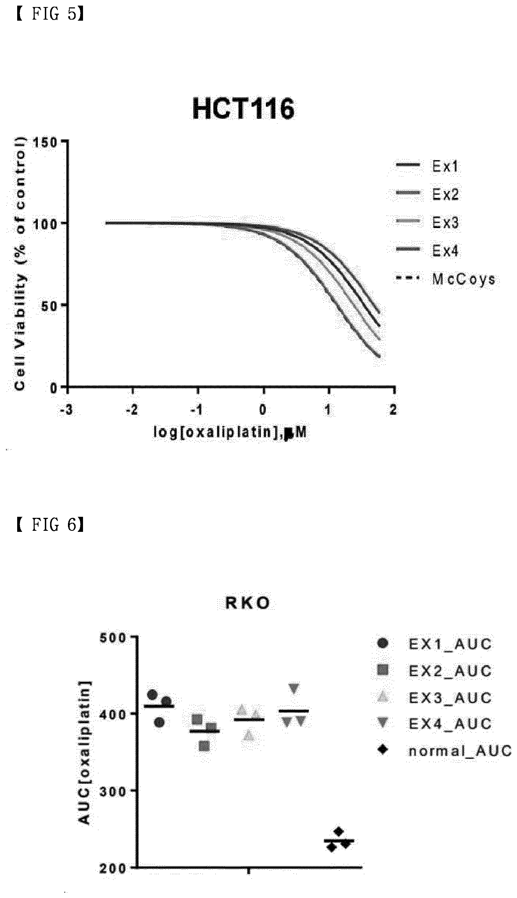

In addition, when treated with oxaliplatin at different concentrations, as shown in FIG. 5, it was confirmed that the cell viability was similarly lowered in the control (McCoys) HCT116 cells and the cells cultured under the organoid culture conditions of Ex2.

### 2-2. Evaluation of Resistance Reduction Effect in RKO Cells

The RKO cell line sensitive to oxaliplatin was used and cultured in the organoid culture media shown in Table 4 above. As a result of treating the RKO cell line and the RKO cells cultured under the conditions of Ex1 to Ex4 with oxaliplatin, as shown in FIG. 6, it was confirmed that the AUC value was the lowest in the organoid culture conditions of Ex2, which do not contain activators and inhibitors, compared to the conditions of Ex1.

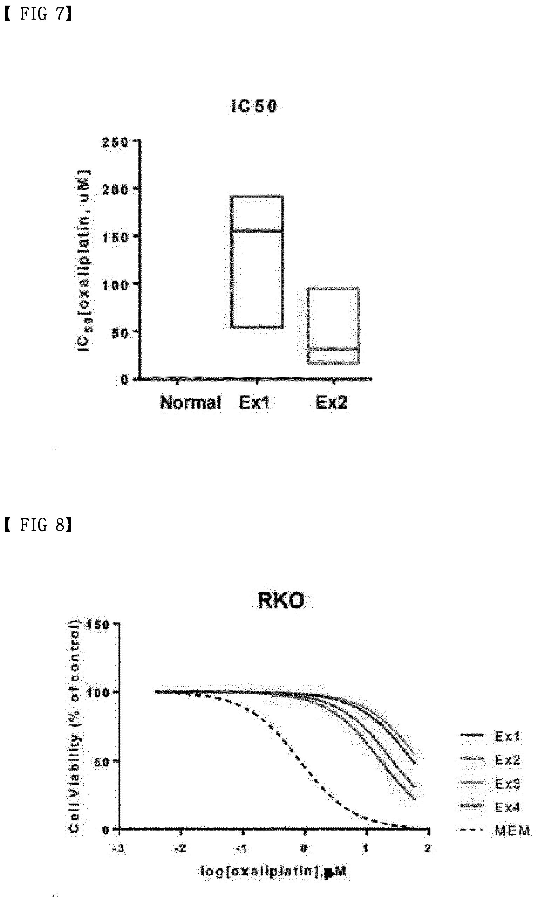

In addition, as shown in FIG. 7, it was confirmed that the IC50 value was significantly lower in Ex2 than in Ex1. As shown in FIG. 8, as a result of treating the cells with oxaliplatin at different concentrations, it was confirmed that the control group RKO cell line (MEM) showed the fastest decrease in cell viability, followed by Ex2.

### 2-3. Evaluation of Resistance Reduction Effect in SW620 Cells

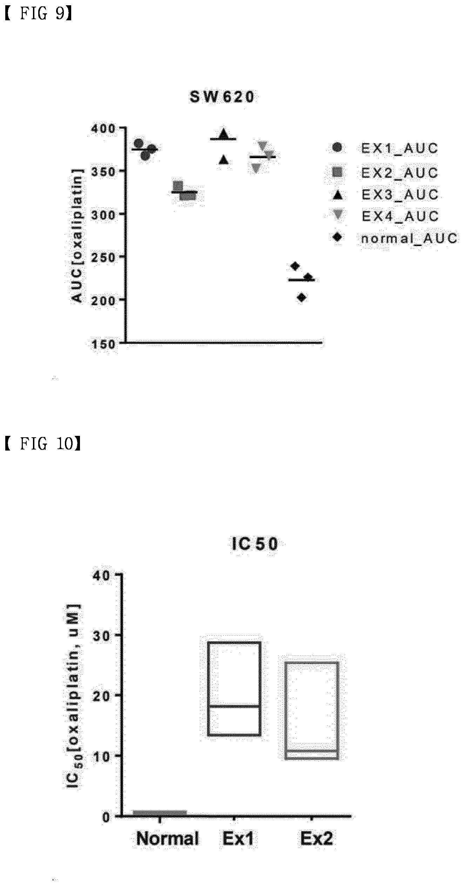

The SW620 cell line sensitive to oxaliplatin was used and cultured in the organoid culture media shown in Table 4 above. As a result of treating the SW620 cell line and the SW620 cells cultured under the conditions of Ex1 to Ex4 with oxaliplatin, as shown in FIG. 9, it was confirmed that the organoid culture conditions Ex3 and Ex4 without activators or inhibitors had no significant difference in AUC values from Ex1 including both activators and inhibitors. However, it was confirmed that the AUC value was lower in the organoid culture conditions of Ex2 without both activators and inhibitors than in other organoid culture conditions.

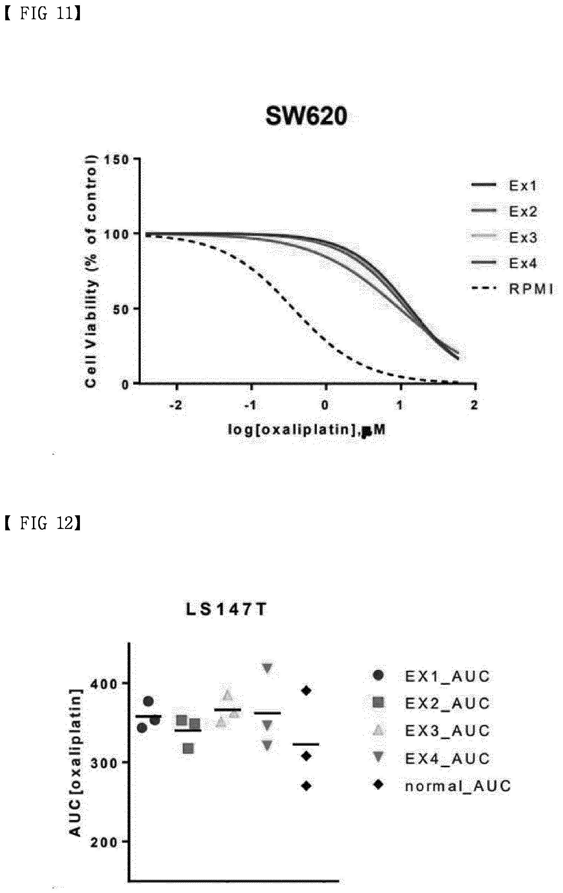

**As** shown in FIG. 10, it was confirmed that the IC50 value was also lower in Ex2 than in Ex1, which shows resistance to oxaliplatin. As shown in FIG. 11, it was confirmed that the cell viability in Ex2 was also lowered to 50% when the log[oxaliplatin] value was 1.

### 2-4. Evaluation of Resistance Reduction Effect in LS147T Cells

The LS147T cell line sensitive to oxaliplatin was used and cultured in the organoid culture media shown in Table 4 above. As a result of treating the LS147T cell line and the LS147T cells cultured under the conditions of Ex1 to Ex4 with oxaliplatin, as shown in FIG. 12, it was confirmed that the organoid culture conditions Ex3 and Ex4 without activators or inhibitors had no difference in AUC values from Ex1 including both activators and inhibitors. However, it was confirmed that the AUC value was lower in the organoid culture conditions of Ex2 without both activators and inhibitors than in the other organoid culture conditions, and that the AUC value in the organoid culture conditions of Ex2 did not significantly differ from the AUC value of the LS147 cell line.

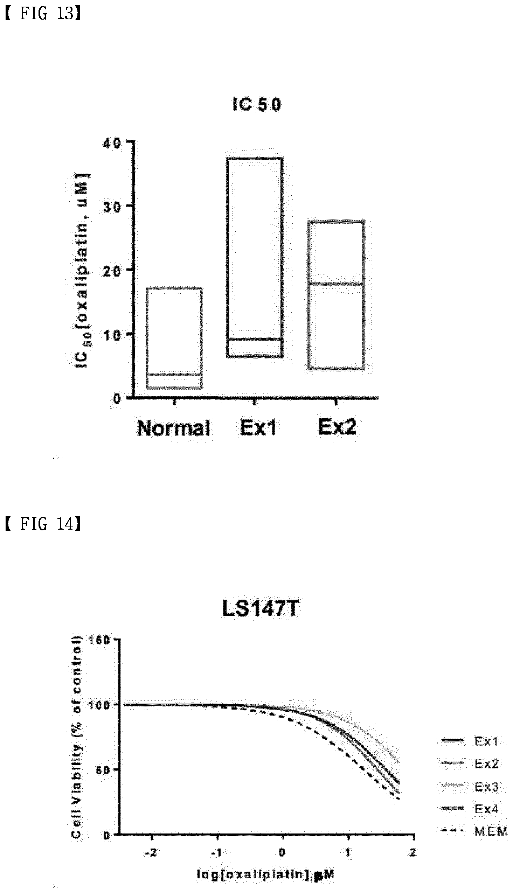

As shown in FIG. 13, it was confirmed that the IC50 value was higher in Ex2 than in Ex1. Therefore, in order to check this point more clearly, as shown in FIG. 14, the cell viability depending on the oxaliplatin treatment concentration was checked. As a result, it was confirmed that the IC50 curve of Ex2 was similar to that of the LS147 cell line.

### 2-5. Evaluation of Resistance Reduction Effect in HT29 Cells

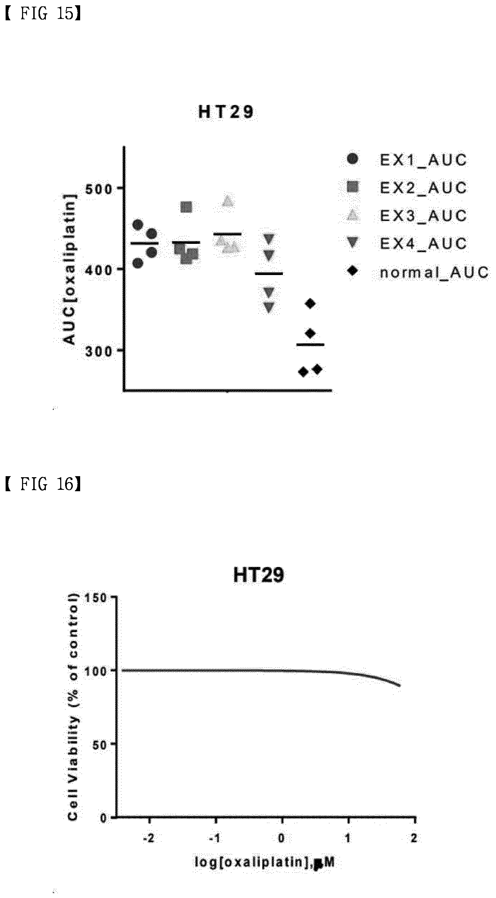

The HT29 cell line not sensitive to oxaliplatin was used and cultured in the organoid culture media shown in Table 4 above. As a result of treating the HT29 cell line and the HT29 cells cultured under the conditions of Ex1 to Ex4 with oxaliplatin, as shown in FIG. 15, it was confirmed that Ex1 to Ex3 showed similar AUC values, but Ex4 showed slightly lower AUC values than Ex1 to Ex3.

As a result of treating the HT29 cell line and the cells cultured under the conditions of Ex1 to Ex4 with oxaliplatin at different concentrations, as shown in FIG. 16, it was confirmed that the cell viability did not decrease under all the culture conditions.

### 2-6. Evaluation of Resistance Reduction Effect in HCT15 Cells

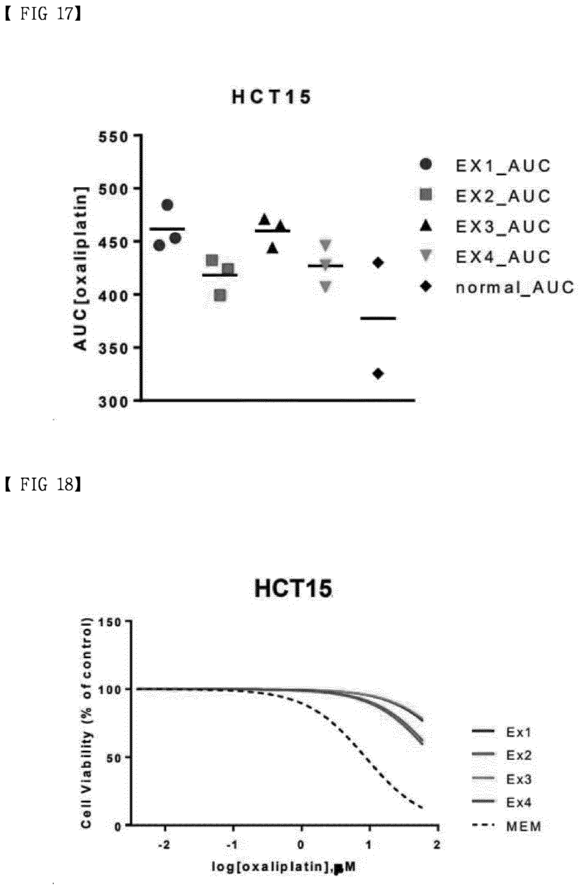

The HCT15 cell line, which is not sensitive to oxaliplatin, was used and cultured in the organoid culture media shown in Table 4 above. As a result of treating the HCT15 cell line and the HCT15 cells cultured under the conditions of Ex1 to Ex4 with oxaliplatin, as shown in FIG. 17, it was confirmed that the AUC value decreased under the organoid culture conditions of Ex2 excluding activators, and the AUC value of Ex4 also decreased compared to those of the other organoids.

To clarify this finding, each organoid and the cell line were treated with oxaliplatin at different concentrations. As a result, as shown in FIG. 18, it was confirmed that, similar to the AUC value, the cell viability in Ex2 and Ex4 tended to decrease compared to that in Ex1 and Ex3 when the log[oxaliplatin] value was 2, but the cell viability did not decrease below 50%.

### 2-7. Evaluation of Resistance Reduction Effect in CoLo205 Cells

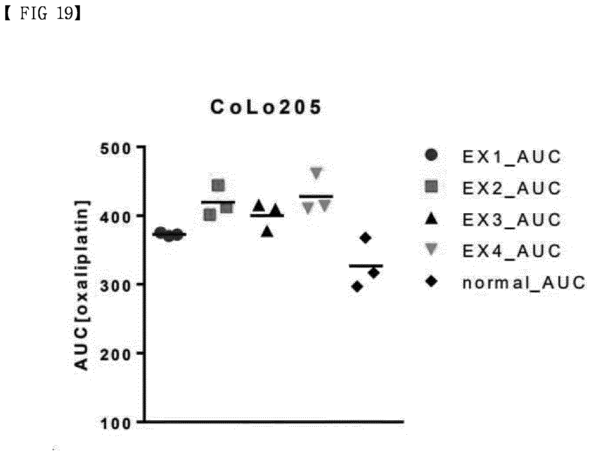

The CoLo205 cell line, which is not sensitive to oxaliplatin, was used and cultured in the organoid culture media shown in Table 4 above. As a result of treating the CoLo205 cell line and the cells cultured under the conditions of Ex1 to Ex4 with oxaliplatin, as shown in FIG. 19, it was confirmed that the AUC values of organoids Ex2 to Ex4 produced without inhibitors or activators were higher than that of Ex1.

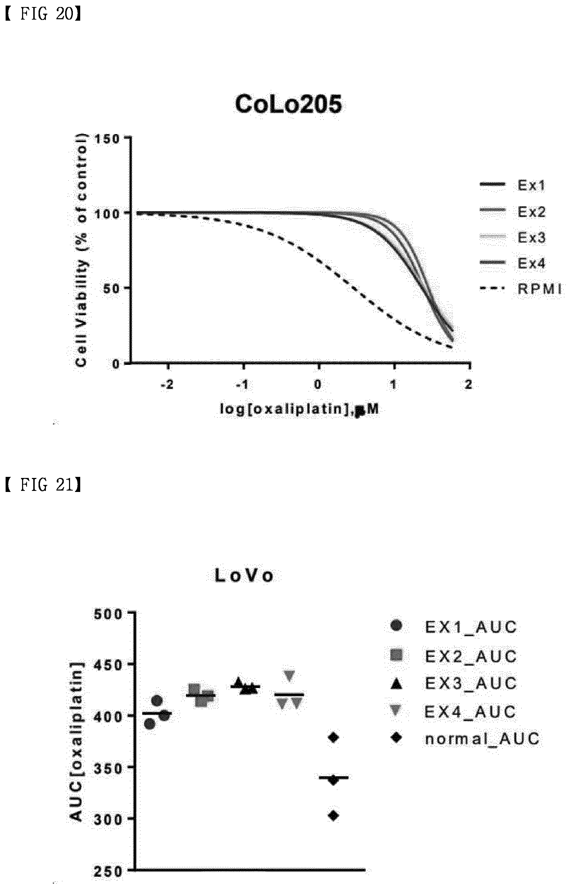

In addition, as shown in FIG. 20, it was confirmed that, when treated with oxaliplatin at different concentrations, the organoids showed similar cell viabilities regardless of the factors contained in the organoids, even though there were some differences.

### 2-8. Evaluation of Resistance Reduction Effect in LoVo Cells

The LoVo cell line, which is not sensitive to oxaliplatin, was used and cultured in the organoid culture media shown in Table 4 above. As a result of treating the LoVo cell line and the LoVo cells cultured under the conditions of Ex1 to Ex4 with oxaliplatin, as shown in FIG. 21, it was confirmed that the AUC values of organoids Ex2 to Ex4 produced without inhibitors or activators were higher than that of Ex1.

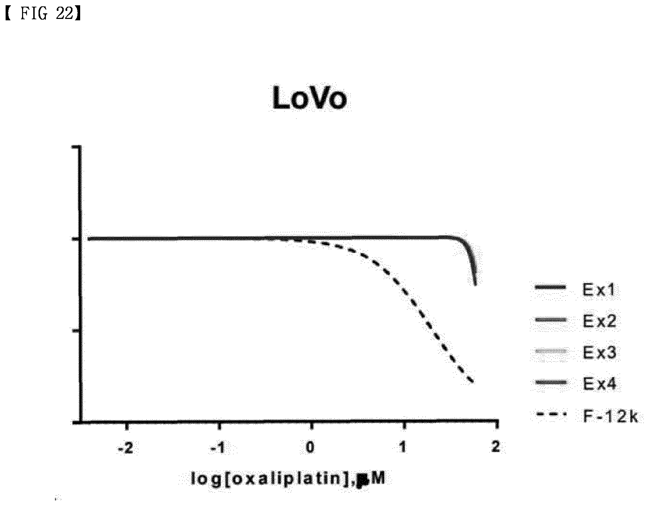

In addition, as a result of checking the cell viability depending on the treatment concentration of oxaliplatin, as shown in FIG. 22, it was confirmed that the log [oxaliplatin] values approached 2, and the cell viabilities of organoids decreased slightly, but did not decrease below 50%.

### 2-9. Comparison of Oxaliplatin Resistance of Ex1 and Ex2 in Colorectal Cancer Cell Lines

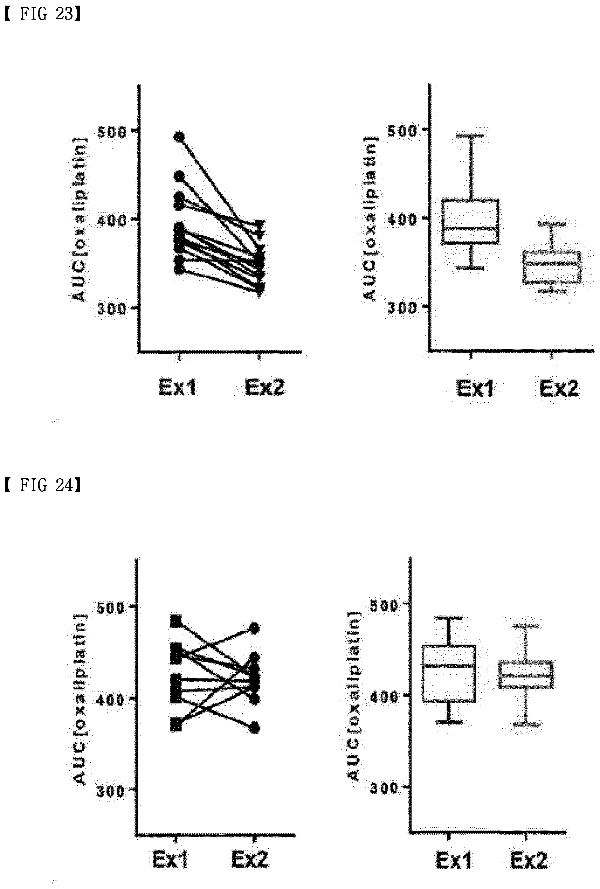

When the above results were combined with the results shown by cell lines with high sensitivity to oxaliplatin and cell lines with low sensitivity to oxaliplatin, the result data shown in FIGS. 23 and 24 and Tables 5 to 7 below were obtained. As shown in FIG. 23 and Table 5 below, it was confirmed that, in the case of cell lines with high sensitivity to oxaliplatin, the sensitivity to oxaliplatin was higher in Ex2 without both inhibitors and activators than in Ex1. On the other hand, as shown in FIG. 24 and Table 6 below, it was confirmed that, in the case of HT29, HTC15, CoLo205, and Lovo cell lines with low sensitivity to oxaliplatin, there was no difference in sensitivity to oxaliplatin between Ex1 and Ex2. Therefore, it was found that, when inhibitors and activators were excluded, resistance to oxaliplatin decreased in cell lines with high sensitivity, while resistance to oxaliplatin did not change in cell lines with low sensitivity.

In addition, as shown in Table 7 below, it was confirmed that the difference in the average value of the total cell line was 23.73, suggesting that sensitivity can be determined based on the difference between the average AUC values of Ex1 and Ex2 of the total cell line.

**[Table 5]**

| Sensitive Cell Line | | |
|---|---|---|
| **normal_AUC** | **EX1_AUC** | **EX2_AUC** |
| 274.55 | 396.08 | 347.78 |
| | EX1-EX2 | 48.30 |

**[Table 6]**

| Resistant Cell Line | | |
|---|---|---|
| **normal_AUC** | **EX1_AUC** | **EX2_AUC** |
| 391.80 | 420.02 | 420.86 |
| | EX1-EX2 | -0.85 |

**[Table 7]**

| Total Cell Line | | |
|---|---|---|
| **normal_AUC** | **EX1_AUC** | **EX2_AUC** |
| 330.83 | 408.05 | 384.32 |
| | EX1-EX2 | 23.73 |

### Example 3. Comparison of Changes in Drug Responsiveness of Ex1 and Ex2 in Colorectal Cancer Cell Lines

HCT-116, a colorectal cancer cell line sensitive to oxaliplatin, and organoids cultured under the Ex1 and Ex2 conditions were treated with oxaliplatin, 5-FU, irinotecan, and folinic acid, and changes in drug responsiveness thereof were compared.

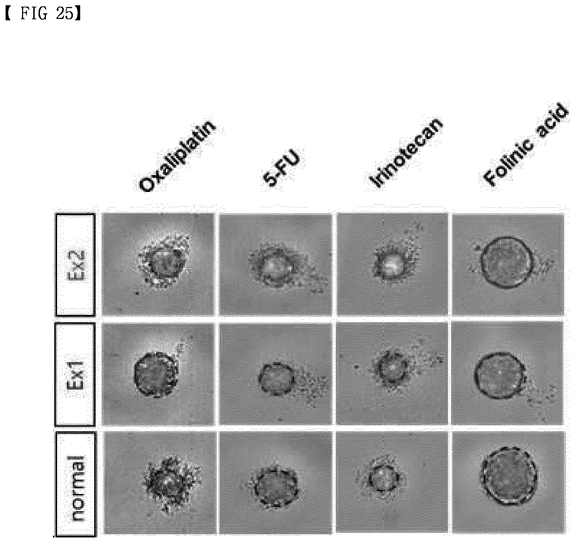

**As** a result, as shown in FIG. 25, it was confirmed that, when treated with 5-FU, irinotecan, and folinic acid, the colorectal cancer cell line showed drug responses similar to the cells cultured in the organoid culture conditions. On the other hand, it was confirmed that, when treated with oxaliplatin, the oxaliplatin resistance was higher in the organoids cultured under the Ex1 conditions than in the colorectal cancer cell line, but the oxaliplatin resistance decreased in Ex2, indicating that the sensitivity to oxaliplatin increased in Ex2.

### Example 4. Evaluation of Differences between 2D Culture and 3D Culture

In order to evaluate the difference between 2D culture and 3D culture performed using the above medium compositions, 166T CoLvM patient-derived organoids were cultured under 2D culture conditions and 3D culture conditions.

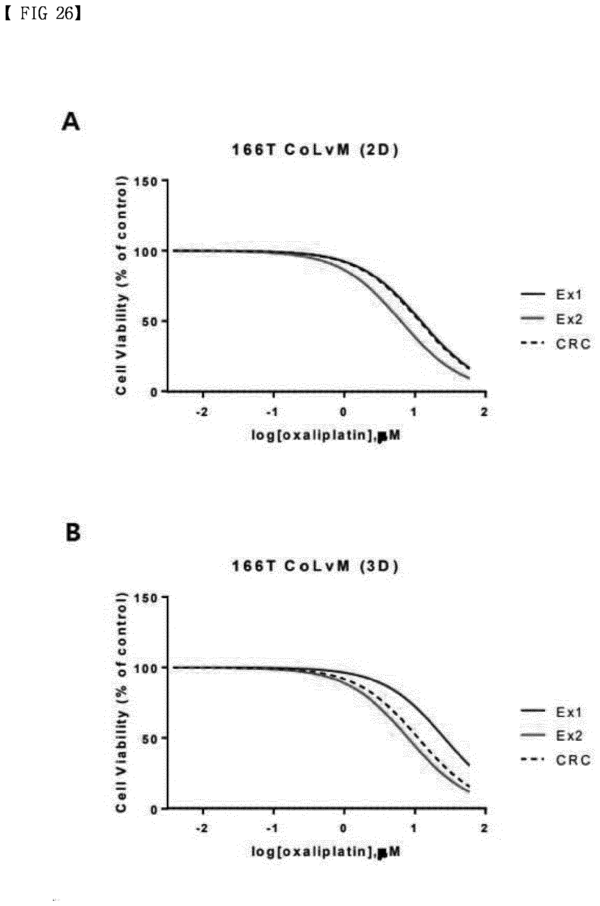

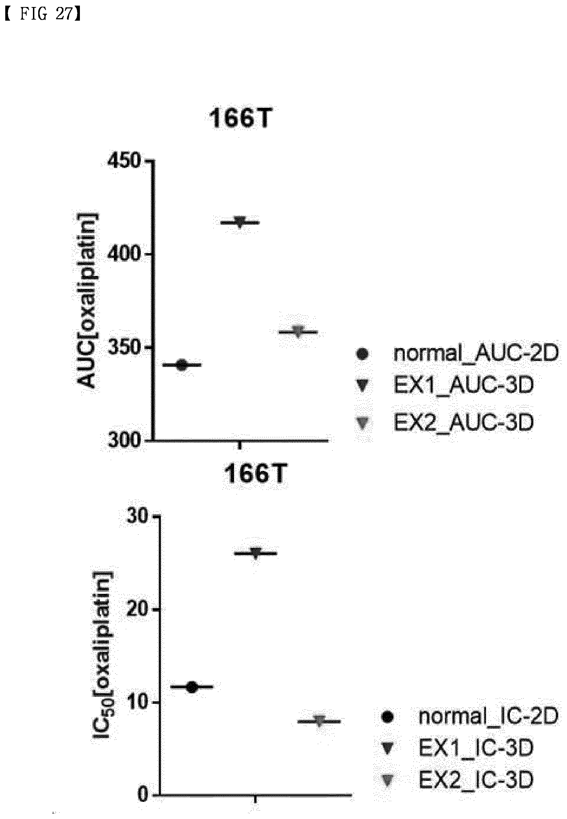

As a result, as shown in FIGS. 26 and 27, it was confirmed that, in 2D culture, the cells cultured in the Ex1 medium showed a cell viability similar to that of the control (CRC) cell line, but in 3D culture, the cells cultured in the Ex1 medium showed a higher cell viability than that of the control (CRC) cell line, indicating that they exhibited resistance to oxaliplatin.

However, it was confirmed that the cells cultured in Ex2 showed cell viabilities similar to those of the control group when 2D and 3D cultured.

### Example 5. Confirmation of Organoid Characteristics

To examine whether organoids produced without inhibitors and activators required for existing organoid culture retain organoid characteristics, the expression of E-cadherin and EpCAM in culture media day 5 after the production of organoids was analyzed by immunofluorescence assay (E-cadherin (24E10) Rabbit mAb (manufacturer: Cell signaling/ 3195S), Alexa Fluor 647 anti-human CD326 (EpCAM) Antibody (manufacturer: Biolegend/324212), Alexa Fluor 488 Goat anti-Rabbit IgG (manufacturer: Invitrogen/A-11008), and Hoechst33342 (manufacturer: Life technologies/H3570) antibody).

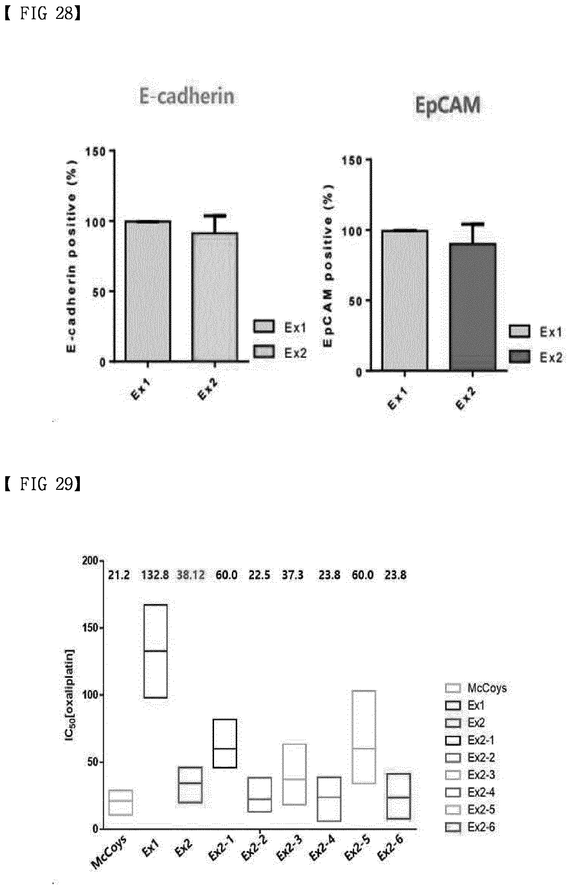

As a result, as shown in FIG. 28, it is confirmed that there was no significant difference in E-cadherin and EpCAM between Ex1 and Ex2, indicating that the organoids produced without both inhibitors and activators retained characteristics as organoids up to day 5.

### Example 6. Identification of Factors That Reduce Resistance to Oxaliplatin

In order to identify the factors that reduce the resistance to oxaliplatin of the Ex2 organoid, activators or inhibitors that change the phenotype were added one by one to the Ex2 organoid composition, thus producing organoids as shown in Table 8 below. Then, it was checked whether the produced organoids had increased resistance to oxaliplatin.

**[Table 8]**

| | **Ex1** | **Ex2** | **Ex2-1** | **Ex2-2** | **Ex2-3** | **Ex2-4** | **Ex2-5** | **Ex2-6** |
|---|---|---|---|---|---|---|---|---|
| **Advanced DMEM/F12** | 1x | 1x | 1x | 1x | 1x | 1x | 1x | 1x |
| **Glutamax** | 1x | 1x | 1x | 1x | 1x | 1x | 1x | 1x |
| **HEPES** | 1x | 1x | 1x | 1x | 1x | 1x | 1x | 1x |
| **Penicillin/ Streptomycin** | 1x | 1x | 1x | 1x | 1x | 1x | 1x | 1x |
| **Primocin** (µg/ml) | 100 | 100 | 100 | 100 | 100 | 100 | 100 | 100 |
| **B27 supplement** | 1x | 1x | 1x | 1x | 1x | 1x | 1x | 1x |
| **N2 supplement** | 1x | 1x | 1x | 1x | 1x | 1x | 1x | 1x |
| **N-Acetylcysteine** (mM) | 1 | 1 | 1 | 1 | 1 | 1 | 1 | 1 |
| **Nicotinamide** (mM) | 10 | 10 | 10 | 10 | 10 | 10 | 10 | 10 |
| **Gastrin** (nM) | 10 | 10 | 10 | 10 | 10 | 10 | 10 | 10 |
| **EGF** (ng/ml) | 10 | 10 | 10 | 10 | 10 | 10 | 10 | 10 |
| **A83-01 (nM)** | 500 | - | 500 | - | - | - | - | - |
| **Y-27632** (µM) | 10 | - | - | 10 | - | - | - | - |
| **SB 202190** (µM) | 10 | - | - | - | 10 | - | - | - |
| **Wnt3a** (µg/ml) | 100 | - | - | - | - | 100 | - | - |
| **R-spondin 1** (µg/ml) | 1 | - | - | - | - | - | 1 | - |
| **Noggin** (ng/ml) | 100 | - | - | - | - | - | - | 100 |

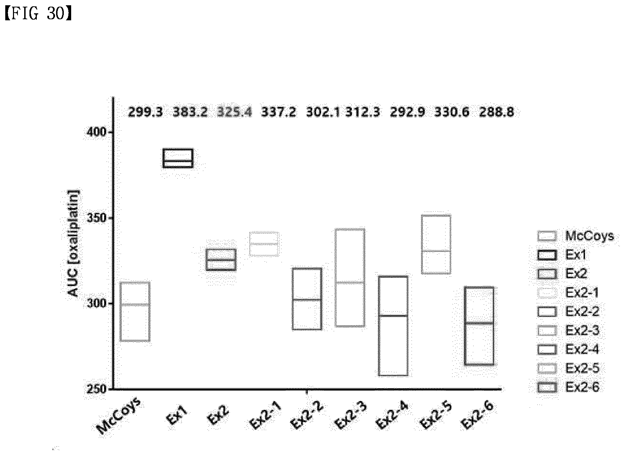

As a result, as shown in FIGS. 29 and 30 and Table 9 below, it was confirmed that, when the cells cultured in Ex2-2, Ex2-4 and Ex2-6 containing one of the activator Y-27632, unlike Ex1, and the inhibitors Wnt3a and Noggin, were treated with oxaliplatin, the AUC and IC50 values thereof were similar to those of the cell line HCT116 (McCoys, control group).

**[Table 9]**

| | **1^{st}** | | **2^{nd}** | | **3^{rd}** | | **Average** | |
|---|---|---|---|---|---|---|---|---|
| | **IC₅₀ (µM)** | **AUC** | **IC₅₀ (µM)** | **AUC** | **IC₅₀ (µM)** | **AUC** | **IC₅₀ (µM)** | **AUC** |
| **McCoys** | 24.1 | 312.3 | 10.7 | 278.4 | 28.8 | 307.3 | 21.2 | 299.3 |
| **Ex1** | 133.0 | 379.7 | 98.0 | 379.9 | 167.3 | 390.0 | 132.8 | 383.2 |
| **Ex2** | 27.8 | 319.8 | 46.1 | 324.8 | 40.5 | 331.7 | 38.1 | 325.4 |
| **Ex2-1** | 46.0 | 328.0 | 52.2 | 341.6 | 81.8 | 342.0 | 60.0 | 337.2 |
| **Ex2-2** | 13.0 | 284.9 | 38.4 | 320.5 | 16.0 | 301.0 | 22.5 | 302.1 |
| **Ex2-3** | 30.2 | 306.6 | 63.4 | 343.3 | 18.2 | 287.0 | 37.3 | 312.3 |
| **Ex2-4** | 5.9 | 258.2 | 38.8 | 304.7 | 26.9 | 315.9 | 23.8 | 292.9 |
| **Ex2-5** | 34.0 | 317.5 | 43.0 | 322.7 | 103.0 | 351.6 | 60.0 | 330.6 |
| **Ex2-6** | 7.8 | 264.3 | 41.4 | 309.4 | 22.1 | 292.6 | 23.8 | 288.8 |

## Claims

1. A composition for predicting efficacy of oxaliplatin drug comprising advanced DMEM/F12, L-glutamine, HEPES, an abtibiotic, B27 supplement, N2 supplement, N-acetylcysteine, nicotinamide, gastrin, and EGF.

2. The composition of claim 1, wherein the antibiotic comprises at least one of penicillin, streptomycin, or Primocin.

3. The composition of claim 1, further comprising at least one of Y-27632, Wnt3a, or Noggin.

4. A method for predicting efficacy of oxaliplatin drug, comprising steps of:
culturing organoids comprising colorectal cancer-derived cells, advanced DMEM/F12, L-glutamine, HEPES, an abtibiotic, B27 supplement, N2 supplement, N-acetylcysteine, nicotinamide, gastrin, and EGF;
treating the cultured organoids with oxaliplatin; and
determining sensitivity to oxaliplatin of the organoids treated with oxaliplatin.

5. The method of claim 4, wherein the organoids in the step of culturing the organoids further comprise at least one of Y-27632, Wnt3a, or Noggin.

6. The method of claim 4, wherein the sensitivity to oxaliplatin is determined by evaluating IC50, AUC, and cell viability.
